# EUROPÄISCHE PATENTSCHRIFT

(11) **EP 1 385 806 B1**
(45) Veröffentlichungstag und Bekanntmachung des Hinweises auf die Patenterteilung: **06.07.2005**
(21) Anmeldenummer: 02735293.9
(22) Anmeldetag: 24.04.2002
(51) Int. Cl.: C07B 31/00, C07C 43/00, C07C 41/00

(54) **EINSTUFIGES VERFAHREN ZUR HERSTELLUNG VON TRIMETHOXYTOLUOL**
SINGLE-STEP METHOD FOR PRODUCING TRIMETHOXYTOLUENE
PROCEDE A UNE ETAPE POUR PRODUIRE LE TRIMETHOXYTOLUENE

(30) Priorität: 27.04.2001 DE 10120911
(43) Veröffentlichungstag der Anmeldung: 04.02.2004
(73) Patentinhaber: BASF AKTIENGESELLSCHAFT, 67056 Ludwigshafen (DE)
(72) Erfinder: FISCHER, Rolf-Hartmuth, 69121 Heidelberg (DE); RÖSCH, Markus, 55276 Oppenheim (DE); GÖTZ, Norbert, 67547 Worms (DE)
(86) Internationale Anmeldenummer: PCT/EP2002/004486
(87) Internationale Veröffentlichungsnummer: WO 2002/088046

(56) Entgegenhaltungen:
- US-A- 2 355 219
- US-A- 2 419 093
- S. LANDA: "Über die Eigenschaften von Sufid-Katalysatoren V. Die Darstellung von Alkylphenolen" COLLECTION OF CZECHOSLOVAK CHEMICAL COMMUNICATIONS., Bd. 23, 1958, Seiten 1322-1329, XP002233768 PRAGUE CS
- H. SUGIHARA: "Synthese von 2.3-Dimethoxy-5-methyl-1.4-benzochinon und seinen Äthylhomologen" LIEBIGS ANNALEN DER CHEMIE, Bd. 763, 1972, Seiten 109-120, XP002233769 WEINHEIM DE in der Anmeldung erwähnt
- DATABASE WPI Section Ch, Week 3081 Derwent Publications Ltd., London, GB; Class E14, AN 1981-54195D XP002233770 & JP 56 068635 A (MITSUI PETROCHEM), 9. Juni 1981 (1981-06-09)

## Beschreibung

Die vorliegende Erfindung betrifft ein Verfahren zur Herstellung von Trimethoxytoluol durch Hydrierung der entsprechenden Benzoesäure und ihren Derivaten, wie Estern oder Anhydriden, mit Wasserstoff in Gegenwart von Hydrierkatalysatoren wie z.B. Cobalt, Nickel-, Ruthenium- oder Palladiumkatalysatoren.

Es ist bekannt, 3.4.5-Trimethoxytoluol dadurch herzustellen, dass man p-Kresol zu 3.5-Dibrom-4-hydroxytoluol bromiert, diese Verbindung mit Natriummethylat zu 3.5-Dimethoxy-4-hydroxytoluol umsetzt und anschließend mit Dimethylsulfat zu 3.4.5-Trimethoxytoluol methyliert (J 5 6068-635, 12.11.1979, Mitsui Petrochemical Ind.). Nachteilig an einem derartigen Verfahren ist, dass das verwendete Brom in Form von Alkalibromid als Koppelprodukt anfällt.

Will man z. B. 3.4.5-Trimethoxytoluol aus Gallussäure (3.4.5-Trihydroxybenzoesäure) oder Derivaten der Gallussäure herstellen, so gelingt die Reduktion der Carboxylgruppe (oder entsprechender Ester) zur Methylgruppe bisher nur in zwei Reaktionsschritten:

Aus Liebigs Annalen der Chemie, Band 763 (1972), Seiten 109 - 120 ist bekannt, Alkylester der 3.4.5-Trialkoxybenzoesäure mit Lithiumaluminiumhydrid zunächst zu 3.4.5-Trialkoxybenzylalkohol zu hydrieren, der dann mit Wasserstoff in Eisessig als Lösungsmittel und Palladium-Aktivkohle-Trägerkatalysatoren zu 3.4.5-Trialkoxytoluol hydriert wird. Nachteilig sind die Zweistufigkeit und der Anfall von Aluminiumoxidhydraten als Koppelprodukt.

In Chemische Berichte Band 99 (1966), Seiten 227 - 230, wird gezeigt, dass sich ausgehend von 3.4.5-Trimethoxybenzoesäure oder Derivaten hergestelltes 3.4.5-Trimethoxybenzonitril mit Terpenen als Wasserstoffdonatoren in Gegenwart von Palladium auf Aktivkohle als Träger zu 3.4.5-Trimethoxytoluol umsetzen lässt. Nachteilig hierbei ist vor allem die Bildung von dehydrierten Terpenen und von Ammoniumsalzen als Koppelprodukten.

Es bestand daher die Aufgabe, ein einstufiges, mit hoher Ausbeute und Selektivität und ohne die Bildung von anorganischen Koppelprodukten verlaufendes Verfahren zur Hydrierung von entsprechend substituierter Benzoesäure, ihren Estern und Anhydriden zu Trimethoxytoluol zu entwickeln.

Diese Aufgabe wird gelöst in einem Verfahren zur Herstellung von Trimethoxytoluol der Formel I 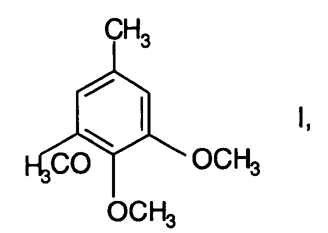 dadurch gekennzeichnet, dass man die entsprechende Benzoesäure, Benzoesäureester oder Benzoesäureanhydride bei geeigneter Temperatur und geeignetem Druck in Gegenwart von homogenen oder heterogenen Katalysatoren, enthaltend
(a) mindestens ein Metall und/oder eine Verbindung auf der Basis eines Metalles (wie beispielsweise Metalloxide, -nitride oder -carbonate), ausgewählt aus der Gruppe, bestehend aus Cobalt, Nickel, Ruthenium und/oder Palladium und
(b) von je 0 bis 30 Gew.-%, bezogen auf die Summe der Komponenten (a) - (c), eines oder mehrerer Metalle oder Verbindungen auf der Basis eines Metalls, ausgewählt aus der Gruppe, bestehend aus Platin, Rhodium, Iridium, Osmium, Kupfer, Eisen, Silber, Gold, Chrom, Molybdän, Wolfram, Mangan, Rhenium, Zink, Cadmium, Blei, Aluminium, Zirkon, Zinn, Phosphor, Silicium, Arsen, Antimon, Bismut und Seltenerdmetalle, sowie
(c) von je 0 bis 5 Gew.-%, bezogen auf die Summe der Komponenten (a) - (c), einer oder mehrerer Verbindungen auf der Basis eines Alkali- oder Erdalkalimetalles,
   wobei die Summe der Komponenten (a) bis (c) 100 Gew.-% beträgt;
mit Wasserstoff umsetzt.

Es gelang nun überraschenderweise, die erfindungsgemäße Umsetzung einstufig und dabei mit hohen Ausbeuten und Selektivitäten an dem Zielprodukt I durchzuführen.

Eine mögliche Ausführungsform des Katalysators enthält
(a) mindestens ein Metall und/oder eine Verbindung auf der Basis eines Metalles (wie beispielsweise Metalloxide, -nitride oder -carbonate), ausgewählt aus der Gruppe, bestehend aus Cobalt, Nickel, Ruthenium und/oder Palladium und
(b) von je 0,1 bis 30 Gew.-%, bezogen auf die Summe der Komponenten (a) - (c), eines oder mehrerer Metalle oder Verbindungen auf der Basis eines Metalls, ausgewählt aus der Gruppe, bestehend aus Platin, Rhodium, Iridium, Osmium, Kupfer, Eisen, Silber, Gold, Chrom, Molybdän, Wolfram, Mangan, Rhenium, Zink, Cadmium, Blei, Aluminium, Zirkon, Zinn, Phosphor, Silicium, Arsen, Antimon, Bismut und Seltenerdmetalle, sowie
(c) von je 0,05 bis 5 Gew.-%, bezogen auf die Summe der Komponenten (a) - (c), einer oder mehrerer Verbindungen auf der Basis eines Alkali- oder Erdalkalimetalles.

Besonders bevorzugte Katalysatoren sind solche, in denen die Komponente (a) mindestens ein Metall und/oder eine Verbindung auf der Basis eines Metalls, ausgewählt aus der Gruppe aus Cobalt und Nickel, in einer Menge im Bereich von je 5 bis 100 Gew.-%. Bevorzugt sind ferner Katalysatoren, in denen die Komponente (a) mindestens ein Metall und/oder eine Verbindung auf der Basis eines Metalles, ausgewählt aus der Gruppe aus Ruthenium und Palladium in einer Menge im Bereich von je 5 bis 100 Gew.-%, jeweils bezogen auf die Summe der Komponenten (a) bis (c), enthält.

Besonders bevorzugte Katalysatoren enthalten als Komponente (b) mindestens ein Metall oder eine Verbindung auf der Basis eines Metalls, ausgewählt aus der Gruppe bestehend aus Silber, Kupfer, Molybdän, Mangan, Rhenium, Blei und Phosphor, in einer Menge im Bereich von je 0 bis 25 Gew.-%, bezogen auf die Summe der Komponenten (a) bis (c).

Besonders bevorzugte Katalysatoren enthalten als Komponente (c) mindestens eine Verbindung auf der Basis der Alkalimetalle und Erdalkalimetalle, ausgewählt aus der Gruppe bestehend aus Lithium, Natrium, Kalium, Cäsium, Magnesium und Calcium, in einer Menge im Bereich von je 0 bis 5 Gew.-%, bezogen auf die Summe der Komponenten (a) bis (c).

Ganz besonders bevorzugte Katalysatoren enthalten nur Komponente (a) und keine Komponenten (b) und (c).

Besonders bevorzugte Katalysatoren enthalten als Komponente (a) Cobalt oder eine Cobalt-Verbindung.

Bevorzugt sind ferner Katalysatoren, die als Komponente (a) Palladium oder eine Palladium-Verbindung enthalten.

Als Ausgangsstoffe können insbesondere Verbindungen der Formel II 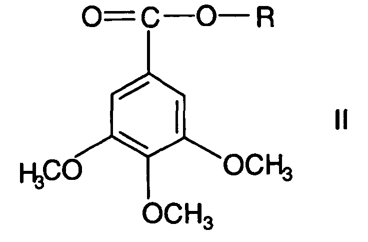 eingesetzt werden, in der R Wasserstoff, Alkylreste mit beispielsweise einem bis zwölf Kohlenstoffatomen, Arylreste, Cycloalkylreste, heterocyclische Reste und/oder Reste -CO-R¹, in denen R¹ Alkylreste mit beispielsweise einem bis sechs Kohlenstoffatomen darstellt, bedeutet.

Geht man von Carbonsäureanhydriden der Formel II aus, in der R -CO-R¹ bedeutet, so sind Katalysatoren besonders bevorzugt, die als Komponente (a) Palladium enthalten.

Die Katalysatoren können als homogene Katalysatoren in gelöster Form oder als heterogene Katalysatoren verwendet werden. Bei den heterogenen Katalysatoren kann es sich um Trägerkatalysatoren, Vollkatalysatoren oder Raneykatalysatoren handeln, die in fest angeordneter, in suspendierter oder in gewirbelter Form angewandt werden. Als Trägermaterialien kommen beispielsweise Oxide wie Aluminiumoxid, Siliciumdioxid, Alumosilikate, Lanthanoxid, Titandioxid, Zirkondioxid, Magnesiumoxid, Zinkoxid und Zeolithe sowie Aktivkohle oder Mischungen davon in Betracht.

Die Herstellung der heterogenen Katalysatoren erfolgt in der Regel derart, dass man Vorläufer der Komponenten (a), optional zusammen mit Vorläufern der Komponenten (b) (Promotoren) und/oder optional mit Vorläufern der Spurenkomponenten (c) in Gegenwart oder Abwesenheit von Trägermaterialien (je nachdem welcher Katalysatortyp gewünscht ist) ausfällt, optional den so erhaltenen Katalysatorvorläufer zu Strängen oder Tabletten verarbeitet, trocknet und anschließend calciniert. Trägerkatalysatoren sind im allgemeinen auch erhältlich, indem man den Träger mit einer Lösung der Komponenten (a) und optional (b) und/oder (c) tränkt, wobei man die einzelnen Komponenten gleichzeitig oder nacheinander zugeben kann, oder indem man die Komponenten (a) und optional (b) und/oder (c) auf den Träger nach an sich bekannten Methoden aufsprüht. Wenn notig, können bei der Katalysator-Herstellung Bindemittel verwendet werden.

Als Vorläufer der Komponenten (a) kommen in der Regel gut wasserlösliche Salze der zuvor genannten Metalle wie Nitrate, Chloride, Acetate, Formiate und Sulfate in Betracht, vorzugsweise Nitrate.

Als Vorläufer der Komponenten (b) kommen in der Regel gut wasserlösliche Salze oder Komplexsalze der zuvor genannten Metalle wie Nitrate, Chloride, Acetate, Formiate und Sulfate in Betracht, vorzugsweise Nitrate.

Als Vorläufer der Komponenten (c) kommen in der Regel gut wasserlösliche Salze der zuvor genannten Alkalimetalle und Erdalkalimetalle wie Hydroxide, Carbonate, Nitrate, Chloride, Acetate, Formiate und Sulfate in Betracht, vorzugsweise Hydroxide und Carbonate.

Die Fällung erfolgt im allgemeinen aus wässrigen Lösungen, wahlweise durch Zugabe von Fällungsreagenzien, durch Änderung des pH-Wertes oder durch Änderung der Temperatur.

Üblicherweise trocknet man die so erhaltene Katalysatorvormasse bei Temperaturen im Bereich von 80 bis 150°C, vorzugsweise von 80 bis 120 °C, vor.

Das Calcinieren nimmt man üblicherweise bei Temperaturen im Bereich von 150 bis 500°C, vorzugsweise von 200 bis 450 °C, in einem Gasstrom aus Luft oder Stickstoff vor.

Gegebenenfalls wird die Katalysator-Oberfläche passiviert, was üblicherweise bei Temperaturen im Bereich von 20 bis 80, vorzugsweise von 25 bis 35°C mittels Sauerstoff-Stickstoff-Mischungen wie Luft durchgeführt wird.

Man setzt die erhaltene calcinierte und gegebenenfalls passivierte Katalysatormasse im allgemeinen einer reduzierenden Atmosphäre aus ("Aktivierung"), beispielsweise indem man sie bei einer Temperatur im Bereich von 80 bis 250°C, vorzugsweise von 80 bis 180 °C, bei Katalysatoren auf der Basis von Ruthenium oder Palladium und/oder Verbindungen auf der Basis von Ruthenium oder Palladium als Komponente (a), oder im Bereich von 150 bis 500°C, vorzugsweise von 180 bis 400°C bei Katalysatoren auf der Basis eines oder mehrerer Metalle und/oder Verbindungen auf der Basis der Metalle ausgewählt aus der Gruppe aus Nickel und Cobalt als Komponente (a) 2 bis 60 h einem Gasstrom, der freien Wasserstoff enthält, aussetzt. Der Gasstrom besteht vorzugsweise aus 20 bis 100 Vol-% Wasserstoff und 0 bis 80 Vol.-% eines Inertgases wie Stickstoff.

Aus der bevorzugten Aktivierung des Katalysators direkt im Synthesereaktor ergeben sich verfahrensökonomische Vorteile.

Die Hydrierung kann diskontinuierlich, insbesondere aber kontinuierlich durchgeführt werden. Beim kontinuierlichen Betrieb kann in Sumpf oder Rieselfahrweise, in der Gas- oder bevorzugt in der Flüssigphase gearbeitet werden.

Die Ausgangsstoffe II können in Substanz, z. B. auch als Schmelzen oder aber in einem Lösungsmittel gelöst, hydriert werden.

Als Lösungsmittel sind solche geeignet, die ein ausreichendes Lösungsvermögen für die Ausgangsstoffe II und die Zielprodukte I aufweisen und die unter den Hydrierbedingungen stabil sind. Beispiele für derartige Lösungsmittel sind Ether wie Tetrahydrofuran, Dioxan, Tetrahydropyran, Polyethylenglykoldialkylether oder Polyethylenglykolmonoalkylether, Alkohole wie Methanol, Ethanol, tert.-Butanol, Cyclohexanol, Wasser, Carbonsäuren, Phenole wie Brenzkatechin, Resorcin, Hydrochinon, Pyrogallol oder Alkylether dieser Phenole.

Bevorzugte Lösungsmittel sind Tetrahydrofuran, Dioxan, Tetrahydropyran, Polyethylenglykoldialkylether, Polyethylenglykolmonoalkylether, Wasser, Essigsäure oder Propionsäure.

Besonders bevorzugte Lösungsmittel sind Wasser, Ether, insbesondere cyclische Ether und Polyethylenglykolmono- oder dialkylether.

Die Hydrierung in Gegenwart von Palladium-Katalysatoren kann auch vorteilhaft in Carbonsäuren wie z. B. C₁-C₄-Carbonsauren wie Essigsäure oder Propionsäure durchgeführt werden.

Hydriert wird z. B. eine 1 bis 60 Gew.-%ige Lösung der Ausgangsstoffe II in den genannten Lösungsmitteln.

Man hydriert bei Temperaturen von wahlweise 20 bis 260°C und Drukken von wahlweise 1 bis 300 bar. In Gegenwart von Palladium- oder Rutheniumkatalysatoren hydriert man bevorzugt bei Temperaturen von 20 bis 150 °C und Drucken von 1 bis 150 bar. In Gegenwart von Nickel- und Cobaltkatalysatoren dagegen bevorzugt bei Temperaturen von 100 bis 260°C und Drucken von 50 bis 300 bar.

Bevorzugt ist die Hydrierung bei höheren Drücken.

Der zur Hydrierung eingesetzte Wasserstoff wird im allgemeinen in größerem stöchiometrischen Überschuss relativ zur Ausgangsverbindung II verwendet.

Er kann als Kreisgas in die Reaktion zurückgeführt werden. Der Wasserstoff kommt im allgemeinen technisch rein zum Einsatz. Beimengen von Inertgasen, z. B. Stickstoff stören jedoch den Reaktionsablauf nicht.

Die durch die erfindungsgemäße Hydrierung herstellbaren Verbindungen I stellen wertvolle Zwischenprodukte dar, die zur Herstellung von Pharmaprodukten, Feinchemikalien und Pflanzenschutzmitteln verwendet werden können.

Nachstehend wird die Erfindung anhand von Ausführungsbeispielen näher erläutert.

### Katalysatoren

Katalysator A: 100% Pd, als 10 % Pd auf Aktivkohle (Sigma-Aldrich Chemie GmbH)
Katalysator B 65,4 % CoO; 20,2 % CuO; 8,3 %Mn₃O₄; 3,5 % MoO₃; 2,4 % P₂O₅; 0,2 % Na₂O
Katalysator C 74,0 % NiO; 2,2 % MoO₃; 23,8 % CuO; auf ZrO₂ als Träger

### Aktivierung der Katalysatoren B und C bei Normaldruck

Nach Füllung eines elektrisch beheizbaren Reaktors von 1 Liter Volumen mit dem Katalysator wird bei einem Stickstoffstrom von 300 l/h die Temperatur ausgehend von Raumtemperatur stündlich um ca. 20°C erhöht, bis 290 °C erreicht sind. Jetzt wird innerhalb von 6 Stunden der Stickstoff gegen Wasserstoff ausgetauscht. Dazu wird stündlich der Wasserstoffanteil um 50 l/h erhöht und gleichzeitig der Stickstoffanteil um 50 l/h verringert. Sind 300 l/h Wasserstoff-Zufuhr erreicht, wird die Reaktortemperatur auf 300-310 °C erhöht und 48 Stunden bei 300 l/h Wasserstoff beibehalten. Der Katalysator wird nach Abkühlung unter Argon ausgebaut und kann unter Tetraethylenglycoldimethylether gelagert werden.

Herstellung von Trimethoxytoluol der Formel I

### Beispiel 1

5,22 g 3.4.5-Trimethoxybenzoesäure-Derivat II (R = -CO-R¹; R¹ = OC₂H₅) werden in 30 ml Eisessig gelöst und mit 0,62 g Kat. A in einen 50 ml Autoklaven gegeben. Bei Raumtemperatur werden 120 bar Wasserstoff aufgepresst. In regelmäßigen Abständen wird Wasserstoff solange nachgepresst, bis Druckkonstanz erreicht wird. Anschließend wird entspannt und im Austrag werden 81 % 3.4.5-Trimethoxytoluol und 4 % 3.4.5-Trimethoxybenzoesäure gefunden.

### Beispiel 2

20 g 3.4.5-Trimethoxybenzoesäure werden in 130 ml Tetraethylenglycoldimethylether gelöst und mit 5 g des aktivierten Katalysators B in einen Autoklaven gegeben. Nach mehrmaligem Spülen mit Wasserstoff werden bei Raumtemperatur 50 bar Wasserstoff aufgepresst, der Autoklaveninhalt auf 180 °C erwärmt und der Wasserstoffdruck auf 200 bar erhöht. In regelmäßigen Abständen wird Wasserstoff solange nachgepresst, bis Druckkonstanz erreicht wird. Im Austrag werden bei einem Umsatz von 97 % 74,6 % 3.4.5-Trimethoxytoluol gefunden.

### Beispiel 3

10 g 3.4.5-Trimethoxybenzoesäure werden in 130 ml Tetrahydrofuran gelöst und mit 2,5 g des aktivierten Katalysators B in einen Autoklaven gegeben. Nach mehrmaligem Spülen mit Wasserstoff werden bei Raumtemperatur 50 bar Wasserstoff aufgepresst, der Autoklaveninhalt auf 180 °C erwärmt und der Wasserstoffdruck auf 200 bar erhöht. In regelmäßigen Abständen wird Wasserstoff solange nachgepresst, bis Druckkonstanz erreicht wird. Im Austrag werden bei einem Umsatz von 73,2 % 48,6 % 3.4.5-Trimethoxytoluol gefunden.

### Beispiel 4

2 g 3.4.5-Trimethoxybenzoesäure werden in 130 ml Wasser gelöst und mit 1 g des aktivierten Katalysators C in einen Autoklaven befüllt. Nach mehrmaligem Spülen mit Wasserstoff werden bei Raumtemperatur 50 bar Wasserstoff aufgepresst, der Autoklaveninhalt auf 180 °C erwärmt und der Wasserstoffdruck auf 200 bar erhöht. In regelmäßigen Abständen wird Wasserstoff solange nachgepresst, bis Druckkonstanz erreicht wird. Im Austrag werden bei einem Umsatz von 68 % 53,3 % 3.4.5-Trimethoxytoluol gefunden.

## Patentansprüche

1. Einstufiges Verfahren zur Herstellung von 3,4,5-Trimethoxytoluol der Formel I 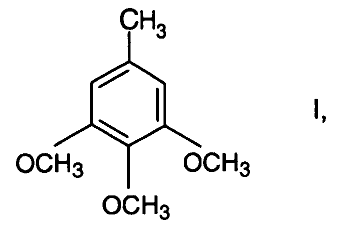 **dadurch gekennzeichnet, dass** man die entsprechenden Benzoesäure, Benzoesäureester oder Benzoeäureanhydride in Gegenwart eines Katalysators enthaltend:
(a) mindestens ein Metall und/oder eine Verbindung auf der Basis eines Metalls, ausgewählt aus der Gruppe, bestehend aus Cobalt, Nickel, Ruthenium und/oder Palladium, und
(b) von je 0 bis 30 Gew.-%, bezogen auf die Summe der Komponenten (a) - (c), eines oder mehrerer Metalle oder Verbindungen auf der Basis eines Metalls, ausgewählt aus der Gruppe, bestehend aus Platin, Rhodium, Iridium, Osmium, Kupfer, Eisen, Silber, Gold, Chrom, Molybdän, Wolfram, Mangan, Rhenium, Zink, Cadmium, Blei, Aluminium, Zirkon, Zinn, Phosphor, Silicium, Arsen, Antimon, Bismut und Seltenerdmetalle, sowie
(c) von je 0 bis 5 Gew.-%, bezogen auf die Summe der Komponenten (a) - (c), einer oder mehrerer Verbindungen auf der Basis eines Alkali- oder Erdalkalimetalles,
wobei die Summe der Komponenten (a) bis (c) 100 Gew.-% beträgt, mit Wasserstoff umsetzt.

2. Verfahren nach Anspruch 1, **dadurch gekennzeichnet, dass** der Katalysator als Komponente (a) mindestens ein Metall und/oder eine Verbindung auf der Basis eines Metalls, ausgewählt aus der Gruppe aus Cobalt und Nickel, in einer Menge im Bereich von je 5 bis 100 Gew.-%.

3. Verfahren nach Anspruch 1, **dadurch gekennzeichnet, dass** der Katalysator als Komponente (a) mindestens ein Metall und/oder eine Verbindung auf der Basis eines Metalls, ausgewählt aus der Gruppe aus Ruthenium und Palladium in einer Menge im Bereich von je 5 bis 100 Gew.-%, jeweils bezogen auf die Summe der Komponenten (a) bis (c), enthält.

4. Verfahren nach Anspruch 1, **dadurch gekennzeichnet, dass** der Katalysator als Komponente (b) ein Metall oder eine Verbindung auf der Basis eines Metalls, ausgewählt aus der Gruppe bestehend aus Silber, Kupfer, Molybdän, Mangan, Rhenium, Blei und Phosphor enthält.

5. Verfahren nach Anspruch 1, **dadurch gekennzeichnet, dass** der Katalysator als Komponente (c) eine Verbindung auf der Basis der Alkalimetalle und Erdalkalimetalle, ausgewählt aus der Gruppe bestehend aus Lithium, Natrium, Kalium, Cäsium, Magnesium und Calcium enthält.

6. Verfahren nach Anspruch 1, **dadurch gekennzeichnet, dass** man Katalysatoren verwendet, die als Komponente (a) Cobalt oder eine Cobalt-Verbindung enthalten.

7. Verfahren nach den Ansprüchen 1 oder 2, **dadurch gekennzeichnet, dass** man beim Einsatz von Benzoesäureanhydrid als Ausgangsverbindung Katalysatoren verwendet, die als Komponente (a) Palladium oder eine Palladium-Verbindung enthalten.

8. Verfahren nach den Ansprüchen 1 bis 7, **dadurch gekennzeichnet, dass** die Hydrierung in der Flüssigphase durchgeführt wird.

9. Verfahren nach den Ansprüchen 1 bis 8, **dadurch gekennzeichnet, dass** man die Hydrierung in einem Lösungsmittel ausgewählt aus Tetrahydrofuran, Dioxan, Tetrahydropyran, Polyethylenglykoldialkylether, Polyethylenglykolmonoalkylether, Methanol, Ethanol, tert.-Butanol, Cyclohexanol, Wasser, Carbonsäuren oder aus den Phenolen Brenzkatechin, Resorcin, Hydrochinon, Pyrogallol oder Alkylethern dieser Phenole durchführt.

10. Verfahren nach den Ansprüchen 1 bis 9, **dadurch gekennzeichnet, dass** man die Hydrierung in einem Lösungsmittel ausgewählt aus Tetrahydrofuran, Dioxan, Tetrahydropyran, Polyethylenglykoldiether, Polyethylenglykolmonoether, Wasser, Essigsäure oder Propionsäure durchführt.

11. Verfahren nach den Ansprüchen 1 bis 10, **dadurch gekennzeichnet, dass** man die Hydrierung bei Drücken von 1 bis 300 bar und bei Temperaturen von 20 bis 260 °C durchführt.

12. Verfahren nach Anspruch 1, **dadurch gekennzeichnet, dass** man die Hydrierung in Gegenwart von Katalysatoren, die als Komponente (a) Nickel oder Cobalt enthalten, bei Drücken von 50 bis 300 bar und bei Temperaturen von 100 bis 260 °C durchführt.

13. Verfahren nach Anspruch 1, **dadurch gekennzeichnet, dass** man die Hydrierung in Gegenwart von Katalysatoren, die als Komponente (a) Palladium oder Ruthenium enthalten, bei Drücken von 1 bis 150 bar und bei Temperaturen von 20 bis 150 °C durchführt.

## Claims

1. A single-stage process for preparing 3,4,5-trimethoxytoluene of the formula I 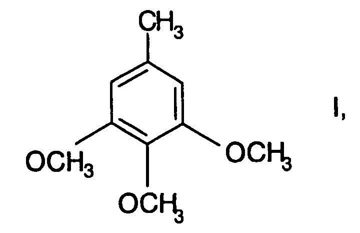 which comprises reacting the corresponding benzoic acid, benzoic esters or benzoic anhydrides with hydrogen in the presence of a catalyst comprising
(a) at least one metal and/or compound of a metal selected from the group consisting of cobalt, nickel, ruthenium and/or palladium, and
(b) from 0 to 30% by weight, based on the sum of the components (a) - (c), of each of one or more metals or compounds of metals selected from the group consisting of platinum, rhodium, iridium, osmium, copper, iron, silver, gold, chromium, molybdenum, tungsten, manganese, rhenium, zinc, cadmium, lead, aluminum, zirconium, tin, phosphorus, silicon, arsenic, antimony, bismuth and rare earth metals, and also
(c) from 0 to 5% by weight, based on the sum of the components (a) - (c), of each of one or more compounds of alkali metals or alkaline earth metals,
where the sum of the components (a) to (c) is 100% by weight.

2. A process as claimed in claim 1, wherein the catalyst comprises as component (a) at least one metal and/or compound of a metal selected from the group consisting of cobalt and nickel, in an amount of from 5 to 100% by weight each.

3. A process as claimed in claim 1, wherein the catalyst comprises as component (a) at least one metal and/or compound of a metal selected from the group consisting of ruthenium and palladium in an amount of from 5 to 100% by weight each, in each case based on the sum of the components (a) to (c).

4. A process as claimed in claim 1, wherein the catalyst comprises as component (b) a metal or a compound of a metal selected from the group consisting of silver, copper, molybdenum, manganese, rhenium, lead and phosphorus.

5. A process as claimed in claim 1, wherein the catalyst comprises as component (c) a compound of an alkali metal or alkaline earth metal selected from the group consisting of lithium, sodium, potassium, cesium, magnesium and calcium.

6. A process as claimed in claim 1, wherein the catalyst comprises cobalt or a cobalt compound as component (a).

7. A process as claimed in claim 1 or 2, wherein a catalyst comprising palladium or a palladium compound as component (a) is used when benzoic anhydride is employed as starting compound.

8. A process as claimed in any of claims 1 to 7, wherein the hydrogenation is carried out in the liquid phase.

9. A process as claimed in any of claims 1 to 8, wherein the hydrogenation is carried out in a solvent selected from among tetrahydrofuran, dioxane, tetrahydropyran, polyethylene glycol dialkyl ethers, polyethylene glycol monoalkyl ethers, methanol, ethanol, tert-butanol, cyclohexanol, water, carboxylic acids, the phenols catechol, resorcinol, hydroquinone, pyrogallol and alkyl ethers of these phenols.

10. A process as claimed in any of claims 1 to 9, wherein the hydrogenation is carried out in a solvent selected from among tetrahydrofuran, dioxane, tetrahydropyran, polyethylene glycol diethers, polyethylene glycol monoethers, water, acetic acid and propionic acid.

11. A process as claimed in any of claims 1 to 10, wherein the hydrogenation is carried out at from 20 to 260°C and pressures of from 1 to 300 bar.

12. A process as claimed in claim 1, wherein the hydrogenation is carried out at from 100 to 260°C and pressures of from 50 to 300 bar in the presence of a catalyst comprising nickel or cobalt as component (a).

13. A process as claimed in claim 1, wherein the hydrogenation is carried out at from 20 to 150°C and pressures of from 1 to 150 bar in the presence of a catalyst comprising palladium or ruthenium as component (a).

## Revendications

1. Procédé de préparation en une étape de 3,4,5-triméthoxytoluène de la formule I : 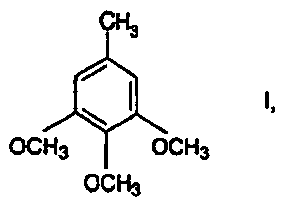 **caractérisé en ce qu'**on fait réagir avec de l'hydrogène l'acide benzoïque, les esters d'acide benzoïque ou les anhydrides d'acide benzoïque correspondants en présence d'un catalyseur contenant :
(a) au moins un métal et/ou un composé à base d'un métal, choisi parmi le groupe constitué de cobalt, nickel, ruthénium et/ou palladium, et
(b) un ou plusieurs métaux ou composés à base d'un métal, choisis parmi le groupe constitué de platine, rhodium, iridium, osmium, cuivre, fer, argent, or, chrome, molybdène, tungstène, manganèse, rhénium, zinc, cadmium, plomb, aluminium, zirconium, étain, phosphore, silicium, arsenic, antimoine, bismuth et métaux des terres rares, chacun à 0 jusqu'à 30% en poids par rapport à la somme des composants (a) - (c), ainsi que
(c) un ou plusieurs composés à base d'un métal alcalin ou alcalino-terreux, chacun à 0 jusqu'à 5% en poids, par rapport à la somme des composants (a) - (c),
la somme des composants (a) à (c) donnant 100% en poids.

2. Procédé suivant la revendication 1, **caractérisé en ce que** le catalyseur contient, comme composant (a), au moins un métal et/ou un composé à base d'un métal, choisi parmi le groupe du cobalt et du nickel, en une quantité de chacun 5 à 100% en poids.

3. Procédé suivant la revendication 1, **caractérisé en ce que** le catalyseur contient, comme composant (a), au moins un métal et/ou un composé à base d'un métal, choisi parmi le groupe du ruthénium et du palladium, en une quantité de chacun 5 à 100% en poids, par rapport à la somme des composants (a) à (c).

4. Procédé suivant la revendication 1, **caractérisé en ce que** le catalyseur contient, comme composant (b), un métal ou un composé à base d'un métal, choisi parmi le groupe constitué d'argent, cuivre, molybdène, manganèse, rhénium, plomb et phosphore.

5. Procédé suivant la revendication 1, **caractérisé en ce que** le catalyseur contient, comme composant (c), un composé à base des métaux alcalins et des métaux alcalino-terreux, choisis parmi le groupe constitué de lithium, sodium, potassium, césium, magnésium et calcium.

6. Procédé suivant la revendication 1, **caractérisé en ce qu'**on utilise des catalyseurs qui contiennent, comme composant (a), du cobalt ou un composé de cobalt.

7. Procédé suivant la revendication 1 ou 2, **caractérisé en ce que**, lors de la mise en oeuvre d'anhydride d'acide benzoïque comme composé de départ, on utilise des catalyseurs qui contiennent, comme composant (a), du palladium ou un composé de palladium.

8. Procédé suivant les revendications 1 à 7, **caractérisé en ce que** l'hydrogénation est effectuée dans la phase liquide.

9. Procédé suivant les revendications 1 à 8, **caractérisé en ce qu'**on effectue l'hydrogénation dans un solvant choisi parmi du tétrahydrofuranne, du dioxanne, du tétrahydropyranne, de l'éther dialkylique de polyéthylèneglycol, de l'éther monoalkylique de polyéthylèneglycol, du méthanol, de l'éthanol, du tert-butanol, du cyclohexanol, de l'eau, des acides carboxyliques ou parmi les phénols, la pyrocatéchine, la résorcine, l'hydroquinone, le pyrogallol ou des éthers alkyliques de ces phénols.

10. Procédé suivant les revendications 1 à 9, **caractérisé en ce qu'**on effectue l'hydrogénation dans un solvant choisi parmi du tétrahydrofuranne, du dioxanne, de tétrahydropyranne, du diéther de polyéthylèneglycol, du monoéther de polyéthylèneglycol, de l'eau, de l'acide acétique ou de l'acide propionique.

11. Procédé suivant les revendications 1 à 10, **caractérisé en ce qu'**on effectue l'hydrogénation à des pressions de 1 à 300 bars et à des températures de 20 à 260°C.

12. Procédé suivant la revendication 1, **caractérisé en ce qu'**on effectue l'hydrogénation en présence de catalyseurs qui, comme composant (a), contiennent du nickel ou du cobalt, à des pressions de 50 à 300 bars et à des températures de 100 à 260°C.

13. Procédé suivant la revendication 1, **caractérisé en ce qu'**on effectue l'hydrogénation en présence de catalyseurs qui, comme composant (a), contiennent du palladium ou du ruthénium, à des pressions de 1 à 150 bars et à des températures de 20 à 150°C.
